# FASCICULE DE BREVET EUROPEEN

(11) **EP 2 183 284 B1**
(45) Date de publication et mention de la délivrance du brevet: **30.05.2018**
(21) Numéro de dépôt: 08846996.0
(22) Date de dépôt: 28.08.2008
(51) Int. Cl.: C08L 5/00, A61Q 5/00, A61Q 5/12, A61Q 7/00, A61Q 7/02, A61Q 9/04, A61Q 17/00, A61Q 19/06, A61Q 19/08, A61K 8/60, A61K 8/73

(54) **UTILISATION COSMÉTIQUE D'UN OU PLUSIEURS COMPOSÉS DE TYPE BETA-(1,3)-GLUCURONANE OU BETA-(1,3)-GLUCOGLUCURONANE COMME AGENT AMINCISSANT OU RAFFERMISSANT**
KOSMETISCHE VERWENDUNG VON EINEM ODER MEHREREN BETA-(1,3)-GLUCURONAN ODER BETA-(1,3)-GLUCOGLUCURONAN ALS SCHLANKHEITSMITTEL ODER STRAFFMITTEL
COSMETIC USE OF ONE OR MORE COMPOUNDS OF THE BETA-(1,3)-GLUCURONANE OR BETA-(1,3)-GLUCOGLUCURONANE TYPE AS SLIMMING AGENT AND TIGHTENING AGENT

(30) Priorité: 31.08.2007 FR 0706121; 27.05.2008 FR 0802869
(43) Date de publication de la demande: 12.05.2010
(73) Titulaire: Greentech, 63360 St Beauzire (FR); Université Clermont Auvergne, 63000 Clermont-Ferrand (FR)
(72) Inventeur: RIOS, Laurent, 63570 Auzat La Combelle (FR); DELATTRE, Cédric, 63110 Beaumont (FR); LAROCHE, Céline, 63350 Crevant-Laveine (FR); MICHAUD, Philippe, 63160 Billon (FR); BERTHON, Jean-Yves, 63540 Romagnat (FR)
(74) Mandataire: Verriest, Philippe
(86) Numéro de dépôt international: PCT/FR2008/001211
(87) Numéro de publication internationale: WO 2009/060140

(56) Documents cités:
- WO-A-91/04988
- WO-A-95/07303
- WO-A-2005/115326
- FR-A- 2 882 366
- US-A1- 2003 209 336

## Description

La présente invention concerne l'utilisation cosmétique d'un ou plusieurs composé(s) de type β-(1,3)-glucuronane ou β-(1,3)-glucoglucuronane comme agent amincissant ou raffermissant.

Les glucuronanes sont des polysaccharides naturels ou des polysaccharides naturels oxydés par voie chimique, contenant des taux significatifs de segments d'acides glucuroniques liés par des liaisons de type β-(1,3), β-(1,4), α-(1,3) ou α-(1,4). A titre d'exemple, on peut citer les glucuronanes bactériens et fongiques (Dantas L. et al., Carbohydr. Res. 1994, 265, 303-310; De Ruiter G.A. et al., Carbohyd. Pol. 1992, 18, 1-7 ; Park J.K. et al., Carbohyd. Pol. 2006, 63, 482-486).

Des études antérieures ont montré que des acides polyglucuroniques pouvaient être obtenus par voies d'oxydations chimiques dites régiosélectives de polysaccharides naturels. Notamment, l'oxydation de polysaccharides par le dioxyde d'azote (NO₂/N₂O₄), comme agent oxydant, est une réaction souvent utilisée. Ainsi, Vignon et al., dans la demande de brevet français FR A 2 873 700, ont décrit une réaction d'oxydation de la cellulose dans un gaz inerte à l'état supercritique à l'aide du dioxyde d'azote comme agent oxydant. Les acides polyglucuroniques obtenus par ce procédé présentent un taux d'acide carboxylique en pourcentage massique allant jusqu'à 25,5% dans le cas de la cellulose. Cependant, ce type de procédés présente les inconvénients inhérents aux manipulations de gaz sous pression. De même, il a été montré que l'usage du réactif TEMPO en milieu hypochloride de sodium permettait aussi l'oxydation regiosélective des fonctions alcool primaire de certains monosaccharides (N. J. Davis and S. L. Flitsch, Tetrahedron Letters 1993, 34(7), 1181-1184). Par un procédé similaire, Vignon et al., dans la demande de brevet internationale WO 03/035699, décrivent l'oxydation par l'utilisation du réactif TEMPO de polyanhydroglucoses estérifiés et plus particulièrement d'esters de cellulose. Le procédé décrit par Vignon nécessite de travailler avec des celluloses estérifiées hydrosolubles ou hydrodispersables. Les produits obtenus par ce procédé présentent de nombreuses applications.

Ainsi, la demande de brevet WO 91/04988 décrit l'utilisation de tels composés comme agent complexant, additif, support, stabilisant ou solubilisant.

La demande de brevet US 2003/0209336 décrit quant à elle l'utilisation de tels composés comme additifs pour papier ou dans une formulation de gels aqueux (contours de l'oeil, amincissants, etc...) sans pour autant mettre en avant l'activité tenseur et/ou raffermissante des composés décrits, seuls leur toxicité, pouvoir d'irritation etc... étant testés.

Ainsi, ces composés n'ont jamais été utilisés ni décrits comme ayant une quelconque activité cosmétique amincissante ou raffermissante.

Or, il a maintenant été découvert, de façon totalement surprenante, que ces composés présentaient des propriétés amincissantes ou raffermissantes. Il a en effet été découvert que certains composés β-(1,3)-glucuronanes ou β-(1,3)-glucoglucuronanes induisent la production et l'organisation de fibrilles de collagène responsable de la réorganisation de la matrice extra-cellulaire. Il a également été découvert que les composés β-(1,3)-glucuronanes ou β-(1,3)-glucoglucuronanes de formule (I) tels que définis selon la présente invention présentent des propriétés cosmétiques amincissantes lorsqu'elles sont appliquées par voie topique ou par voie générale. En effet, ces composés entrainent la surexpression du gêne ANGPTL4 qui code pour l'adipokine qui posséde notamment la propriété d'inhiber l'activité de l'enzyme lipoprotéine lipase (LPL) qui est fixée sur l'endothélium vasculaire accolé aux tissus adipeux. L'inhibition enzymatique empêche le transfert des acides gras depuis les lipoprotéines vers les adipocytes diminuant ainsi fortement la lipogénèse. De plus, l'adipokine possède la propriété d'activer l'enzyme ATGL (Adipose Triglyceride Lipase), présente dans les adipocytes. Cette activation enzymatique augmente ainsi fortement la lipolyse et la libération des acides gras des adipocytes.

La présente invention a donc pour objet l'utilisation de β-(1,3)-glucuronane ou β-(1,3)-glucoglucuronane choisi parmi :
- l'acide β-(1,3)-D-polyglucuronique ayant des masses molaires comprises entre 500 et 2 500 000 Daltons ;
- l'acide β-(1,3)-D-polyglucuronique peracétylé ayant des masses molaires comprises entre 600 et 3 500 000 Daltons; et
- l'acide β-(1,3)-D-polyglucuronique, sulfaté en positions C2 et C4 ayant des masses molaires comprises entre 800 et 4 000 000 Daltons ;
comme agent amincissant ou raffermissant.

La présente invention a également pour objet l'utilisation d'une composition cosmétique comprenant, à titre de principe actif, un ou plusieurs β-(1,3)-glucuronane ou β-(1,3)-glucoglucuronane choisi parmi :
- l'acide β-(1,3)-D-polyglucuronique ayant des masses molaires comprises entre 500 et 2 500 000 Daltons ;
- l'acide β-(1,3)-D-polyglucuronique peracétylé ayant des masses molaires comprises entre 600 et 3 500 000 Daltons; et
- l'acide β-(1,3)-D-polyglucuronique, sulfaté en positions C2 et C4 ayant des masses molaires comprises entre 800 et 4 000 000 Daltons ;
comme composition amincissante ou raffermissante.

Dans le cadre de la présente invention, les expressions « degré d'oxydation » et « poids moléculaire » utilisés ci-après se réfèrent indifféremment à la molécule seule ou au mélange de molécules et représentent alors dans ce cas une valeur moyenne.

Les composés de type β-(1,3)-glucuronanes et/ou β-(1,3)-glucoglucuronanes tels que définis ci-avant peuvent être préparés selon des procédés bien connus de l'homme du métier. A titre d'exemple de tels procédés, on peut citer le procédé de préparation à partir d'au moins un β-(1,3)-glucane linéaire, non ramifié et non estérifié, comprenant au moins une opération d'oxydation régiosélective en position C6 desdits β-(1,3)-glucanes à l'aide de 2,2,6,6-tétraméthylpipéridine-1-oxyle (TEMPO) et d'au moins un co-additif hypochlorite de métal alcalin.
De façon préférentielle, ladite étape d'oxydation régio-sélective du procédé selon la présente invention, est effectuée à l'aide de 2,2,6,6-tétraméthylpipéridine-1-oxyle (TEMPO), et à l'aide d'un co-additif hypochlorite de métal alcalin associé à un bromure de métal alcalin. De façon préférentielle encore, le co-additif hypochlorite de métal alcalin est l'hypochlorite de sodium et le bromure de métal alcalin est le bromure de sodium.

Ledit β-(1,3)-glucane linéaire, non ramifié et non estérifié mis en oeuvre pour réaliser ledit procédé sera ci-après dénommé par « substrat ».

Ledit substrat peut être un β-(1,3)-glucane linéaire, non ramifié et non estérifié obtenu par synthèse ou hémisynthèse, ou bien être d'origine naturelle, de préférence bactérienne. Ledit substrat peut présenter une masse molaire en poids de l'ordre de 500 à 2 500 000 Daltons.

De façon préférentielle, ledit substrat est choisi parmi les β-(1,3)-glucanes linéaires, non ramifiés et non estérifiés, hydrosolubles (solubilité d'au moins 1 g/l dans l'eau de pH alcalin) ou ne formant pas d'agrégat dans le temps.
Un substrat préféré est le curdlane, un β-(1,3)-glucane linéaire, non ramifié d'une taille moléculaire d'environ 2 000 000 à 2 500 000 Daltons, et produit notamment par les bactéries du genre Agrobacterium et Rhizobium.
L'étape d'oxydation sélective est généralement opérée selon la méthode décrite dans la demande de brevet FR A 2 831 171.

Différents degrés d'oxydation des β-(1,3)-glucoglucuronanes préparés par ledit procédé peuvent être obtenus en contrôlant, par exemple, le temps de réaction de ladite étape d'oxydation sélective. En particulier, une oxydation complète du substrat peut être obtenue. Ainsi, ladite réaction d'oxydation sélective peut être arrêtée, par exemple, par addition d'un acide minéral tel que les acides chlorhydrique ou sulfurique. Les β-(1,3)-glucuronanes et/ou β-(1,3)-glucoglucuronanes peuvent être éventuellement précipités au moyen d'un alcool comme l'éthanol. Les β-(1,3)-glucuronanes et/ou β-(1,3)-glucoglucuronanes obtenus à la suite de l'étape d'oxydation peuvent être éventuellement purifiés par toute méthode connue habituelle, par exemple resolubilisation, lavage, dialyse, ou nano et ultrafiltration tangentielle.

Afin de moduler les propriétés des β-(1,3)-glucuronanes et/ou β-(1,3)-glucoglucuronanes obtenus, ladite étape d'oxydation sélective dudit procédé peut être suivie des deux étapes suivantes, dans un ordre quelconque :
a) une opération de fonctionnalisation chimique par des fonctions choisies parmi: methyle, methyloxy, ethyle, ethyloxy, butyl, butyloxy, isobutyle, isobutyloxy, propyl, propyloxy, isopropyle, isopropyloxy, sulfate, sulfoacetate, acetate, propionate, acetobutyrate, acetopropionate, glycerate ou phosphoryl ;
b) une opération de dégradation chimique et/ou physique et/ou enzymatique.

De façon préférentielle, ladite étape d'oxydation sélective dudit procédé peut être suivie des deux étapes suivantes, dans un ordre quelconque :
c) une opération de fonctionnalisation chimique par des fonctions choisies parmi: methyle, methyloxy, ethyle, ethyloxy, butyl, butyloxy, sulfate, sulfoacetate, ou phosphoryl ;
d) une opération de dégradation chimique et/ou physique et/ou enzymatique.

Lesdites étapes peuvent être effectuées suivant les méthodes connues. Les β-(1,3)-glucuronanes et/ou β-(1,3)-glucoglucuronanes intermédiaires et/ou finaux obtenus à la suite de l'une quelconque des étapes ou des deux étapes dudit procédé peuvent être éventuellement purifiés par toute méthode connue habituelle, par exemple resolubilisation, lavage, élimination des sels par dialyse, nano- ou ultrafiltration tangentielle.

Les β-(1,3)-glucuronanes et/ou β-(1,3)-glucoglucuronanes tels que définis ci-avant ou les produits intermédiaires obtenus par le procédé décrit dans le cadre la présente invention peuvent être purifiés par les méthodes connues habituelles, par exemple par resolubilisation, lavage, dialyse ou nano- ou ultrafiltration tangentielle.

Les compositions utilisées dans le cadre de la présente invention peuvent être formulées sous différentes formes galéniques : crèmes, gels, lotions, laits, émulsions huile dans eau ou eau dans huile, solutions, onguents, pulvérisateurs, huiles corporelles, lotions capillaires, shampooings, lotions après-rasage, savons, bâtons protecteur des lèvres, bâtons et crayons pour maquillage à des teneurs de 0,01% à 75% en poids, préférentiellement entre 0,01% et 25% sous forme de poudre et à des teneurs comprises entre 0,01% et 35%, préférentiellement entre 0,01% et 15% sous forme encapsulée.

Pour la préparation de ces compositions, un ou plusieurs composés de type β-(1,3)-glucuronane ou β-(1,3)-glucoglucuronane selon l'invention sont mélangés aux excipients généralement employés dans la technique cosmétique.

Les compositions utilisées dans le cadre la présente invention peuvent prendre la forme d'une crème dans laquelle un ou plusieurs composés de type β-(1,3)-glucuronane ou β-(1,3)-glucoglucuronane sont associés aux excipients couramment utilisés dans la cosmétologie.

Les compositions utilisées dans le cadre la présente invention peuvent prendre la forme de gels dans les excipients appropriés tels que les esters de cellulose ou d'autres agents gélifiants, tels que le carbopol, la gomme guar, etc.

Les compositions utilisées dans le cadre la présente invention peuvent aussi prendre la forme d'une lotion ou d'une solution dans lesquelles un ou plusieurs composés de type β-(1,3)-glucuronane ou β-(1,3)-glucoglucuronane sont sous forme encapsulée.

Les microsphères peuvent par exemple être constituées de corps gras, d'agar et d'eau. Un ou plusieurs composés de type β-(1,3)-glucuronane ou β-(1,3)-glucoglucuronane peuvent être incorporés dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.

Les compositions utilisées dans le cadre de la présente invention peuvent aussi contenir des additifs ou des adjuvants usuels en cosmétologies, comme par exemple des agents antimicrobiens ou des parfums mais aussi des lipides d'extraction ou de synthèse, des polymères gélifiants et viscosants, des tensio-actifs et des émulsifiants, des principes actifs hydro- ou liposolubles, des extraits de plantes, des extraits tissulaires, des extraits marins, des actifs de synthèse.

Les compositions utilisées dans le cadre de la présente invention peuvent aussi comprendre d'autres principes actifs complémentaires choisis pour leur action, par exemple pour l'effet amincissant, l'effet anti-cellulite, l'effet raffermissant, l'effet hydratant, l'activité antimicrobienne, l'activité anti-oxydante, l'activité antiradicalaire, l'effet cicatrisant, l'effet tenseur, l'effet anti-ride, l'activité chélatante, l'activité complexante et séquestrante, l'effet apaisant, l'effet anti-cernes, l'effet anti-rougeurs, l'activité émolliente, l'effet démêlant capillaire, l'activité anti-pelliculaire, l'effet stimulant de la repousse du cheveu, l'effet gainant capillaire, l'activité épilatoire, l'activité limitant la repousse du poil, l'activité participant au renouvellement cellulaire, l'activité modulant la réponse inflammatoire, l'activité participant au maintien de l'ovale du visage, mais également la protection solaire, l'activité anti-irritante, la nutrition cellulaire, la respiration cellulaire, les traitements anti-séborrhéiques, la tonicité cutanée, la protection du cheveu.

Lorsque les compositions utilisées dans le cadre de la présente invention contiennent des principes actifs complémentaires, ceux-ci sont généralement présents dans la composition à une concentration suffisamment élevée pour qu'ils puissent exercer leur activité.

Les compositions utilisées dans le cadre de la présente invention sont de préférences à utiliser quotidiennement en les appliquant une ou plusieurs fois par jour.

Les compositions utilisées dans le cadre de la présente invention sont très bien tolérées, elles ne présentent aucune toxicité et leur application sur la peau, pour des périodes de temps prolongées, n'implique aucun effet systématique.

La présente invention est illustrée de manière non limitative par les exemples suivants.

### Exemple 1 - Exemple de préparation de composés β-(1,3)-glucuronanes ou β-(1,3)-glucoglucuronanes

Les spectres RMN ¹³C ont été enregistrés sur un spectromètre RMN Avance 400 Bruker (9.4 T, 400 MHz). 30000 accumulations ont été réalisées à 30°C.

### Production en milieu liquide de β-(1,3)-glucuronane (degré d'oxydation d'au moins 95%).

Le curdlane (10g) est dissous dans de l'eau distillée (1L) à une température de 0°C et à pH 10. Du TEMPO (43,3 mg), du NaBr (0,95 g) et du NaClO (50 mL à 13 %) sont ajoutés au milieu réactionnel. La réaction d'oxydation est stoppée après 1 heure par ajout de méthanol puis, neutralisée avec de l'acide chlorhydrique (2M). Le *β-*(1,3)-glucuronane formé est précipité avec de l'alcool glacé. Ce polysaccharide anionique est ensuite récupéré par centrifugation (15000 g, 20', 4°C) puis enfin séché par lyophilisation (24h).

L'analyse RMN ¹³C met en évidence l'oxydation complète du carbone C6 du curdlane puisque un signal à 61,4 ppm correspondant au carbone C6 portant l'alcool primaire (CH₂OH) a entièrement disparu et qu'un nouveau signal à 175,5 ppm apparait, caractéristique de la carboxylation du carbone C6 du curdlane (COO⁻). Le degré d'oxydation est également calculé par dosage conductimétrique.

### Production en milieu liquide de β-(1,3)-glucoglucuronane (41% d'oxydation).

Le curdlane (10g) est dissous dans de l'eau distillée (1L) à une température de 0°C et à un pH 10. Du TEMPO (43,3 mg), du NaBr (0,95 g) et du NaClO (50 mL à 13 %) sont ajoutés au milieu réactionnel. La réaction d'oxydation est stoppée après 30 minutes par ajout de méthanol puis, neutralisée avec de l'acide chlorhydrique (2M). Le β-(1,3)-glucoglucuronane formé est précipité avec de l'alcool glacé. Ce polysaccharide partiellement oxydé est ensuite récupéré par centrifugation (15000 g, 20', 4°C) puis enfin séché par lyophilisation (24h).

L'analyse RMN ¹³C met en évidence l'oxydation partielle (41%) du carbone C6 du curdlane par intégration des signaux à 175,5 ppm et 61,4 ppm.

### Dégradation thermique du β-(1,3)-glucuronane.

Une solution de β-(1,3)- glucuronane (10g/L dans l'eau) est chauffée à une température de 100 °C pendant 20 minutes. Après refroidissement, la solution est acidifiée (pH 2) puis centrifugée (15000 g, 20', 4°C). Le surnageant contenant les oligosaccharides est récupéré, neutralisé puis séché par lyophilisation.

### Peracétylation du β-(1,3)-glucuronane

Une solution de β-(1,3)-glucuronane (10g/L dans la pyridine), à laquelle est ajoutée de l'anhydride acétique (1 volume), est maintenue à température ambiante durant une période d'au moins 24 heures (24-48 heures) puis évaporée sous vide. Le β-(1,3)-glucuronane peracétylé ainsi obtenus est récupéré.

L'analyse RMN ¹³C de ces molécules met en évidence la présence d'un signal à 23,4 ppm, caractéristique du groupement méthyl de l'acétate, confirmant ainsi l'acétylation de l'acide β-(1,3)-polyglucuronique.

### Persulfatation du β-(1,3)-glucuronane

Une solution de β-(1,3)-glucuronane (10g/L dans le DMF), à laquelle est ajoutée une solution de SO₃/DMF (1 volume), est maintenue à température ambiante durant une période d'au moins 24 heures (24 à 48 heures) puis neutralisée avant d'être lyophilisée. Le taux de sulfatation est mesuré par dosage potentiométrique.

### Exemple 2 : Activités cosmétiques de composés β-(1,3)-glucuronanes et β-(1,3)-glucoglucuronanes

Les activités biologiques de composés β-(1,3)-glucuronanes ou d'un mélange de composés β-(1,3)-glucoglucuronanes ont été criblées par une méthode originale de biologie moléculaire (transcriptomique) puis mises en évidence par des méthodologies classiquement employées en cosmétologie.

### Exemple 2.1 : Activités amincissante, anti-cellulite, anti- effet peau d'orange d'un composé β-(1,3)-glucuronane ou d'un mélange de composés β-(1,3)-glucoglucuronanes

L'étude transcriptomique réalisée sur des fibroblastes humains cultivés *in-vitro* en absence ou en présence d'un ou plusieurs de ces composés a montré que ces molécules entraînent la surexpression du gène ANGPTL4 qui code pour l'adipokine du même nom. Cette adipokine produite localement au niveau du tissu adipeux inhibe l'activité de l'enzyme lipoprotéine lipase (LPL) empêchant ainsi le transfert des acides gras depuis les lipoprotéines vers les adipocytes diminuant ainsi fortement la lipogénèse. En même temps, cette adipokine active l'enzyme ATGL (Adipose Triglyceride Lipase), présente dans les adipocytes, augmentant ainsi fortement la lipolyse et la libération des acides gras des adipocytes.
Ces résultats ont été complétés par une étude *in-vitro,* réalisée sur des adipocytes cultivés en absence ou en présence d'un composé β-(1,3)-glucuronane ou d'un mélange de composés β-(1,3)-glucoglucuronanes, à la concentration de 0,5%, qui a permis de montrer que ces molécules induisent une diminution de l'ordre de 25% de la densité en acides gras stockés sous forme de triglycérides dans les vacuoles lipidiques des adipocytes.
De plus, une seconde étude *in-vitro,* réalisée sur des pré-adipocytes, a montré que l'utilisation d'un ou plusieurs de ces composés (à la concentration de 0,5%) diminue(nt) fortement la différenciation des pré-adipocytes en adipocytes matures (diminution de l'expression du marqueur aP2/FABP4).
La diminution de la lipogénèse, l'augmentation de la lipolyse, l'augmentation de la libération des acides gras depuis les adipocytes, l'augmentation de l'utilisation de ces acides gras et la diminution de la différenciation des pré-adipocytes en adipocytes matures induites par le composé β-(1,3)-glucuronane ou le mélange de composés β-(1,3)-glucoglucuronanes mettent en évidence l'activité amincissante de ces molécules.

Une étude clinique, réalisée sur des volontaires humains, a montré que l'utilisation quotidienne, en topique, durant 30 jours d'une préparation cosmétique sous forme de crème contenant 3% d'un composé (β-(1,3)-glucuronane ou 3% d'un mélange de composés β-(1,3)-glucoglucuronanes, entraîne une diminution significative de la cellulite chez plus de 63% de ces individus. L'effet peau d'orange est diminué chez 53% des volontaires (contre 27% dans le groupe de volontaires traités avec un placébo).

### Exemple 2.2 : Effet sur le remodelage tissulaire d'un composé β-(1,3)-glucuronane, ou d'un mélange de composés β-(1,3)-glucoglucuronanes

L'étude transcriptomique réalisée sur des fibroblastes humains cultivés *in-vitro* en absence ou en présence d'un ou plusieurs de ces composés a montré que ces molécules entraînent la surexpression des gènes MMP, EGR1, WNT11, PDGFD, TFPI2, PTGS2 et la sous expression du gène HAPLN1 codant pour les protéines portant les mêmes noms et impliquées dans les processus de cicatrisation et de remodelage tissulaire. En effet, l'induction transitoire (sous le contrôle de TFPI2 et de PDGFD) des métaloprotéinases (MMP) et l'inhibition transitoire (sous le contrôle de TFPI2) de HAPLN1, permettent une déstabilisation transitoire et localisée au niveau du site agressé (à cicatriser) de la matrice extracellulaire principalement par une action catabolique sur les fibres de collagène. A la suite de la dégradation de la matrice extracellulaire, contrôlée par TFPI2, ayant permis l'élimination des fibres de collagène lésées et la migration et la prolifération (contrôlées par PTGS2) des cellules indispensables au processus de remodelage tissulaire (fibroblastes, kératinocytes, mélanocytes, etc.), les protéines EGR1 et WNT11 permettent la stimulation de la synthèse des fibres de collagène et donc la reconstruction et la stabilisation de la nouvelle matrice extracellulaire. Donc, via l'induction de l'expression de ces protéines, le composé β-(1,3)-glucuronane ou le mélange de composés β-(1,3)-glucoglucuronanes ont un effet sur le remodelage tissulaire utilisables en cosmétique, notamment dans les produits raffermissants.

## Revendications

1. Utilisation de β-(1,3)-glucuronane ou β-(1,3)-glucoglucuronane choisi parmi :
- l'acide β-(1,3)-D-polyglucuronique ayant des masses molaires comprises entre 500 et 2 500 000 Daltons ;
- l'acide β-(1,3)-D-polyglucuronique peracétylé ayant des masses molaires comprises entre 600 et 3 500 000 Daltons; et
- l'acide β-(1,3)-D-polyglucuronique, sulfaté en positions C2 et C4 ayant des masses molaires comprises entre 800 et 4 000 000 Daltons ;
comme agent amincissant.

2. Utilisation de β-(1,3)-glucuronane ou β-(1,3)-glucoglucuronane choisi parmi :
- l'acide β-(1,3)-D-polyglucuronique ayant des masses molaires comprises entre 500 et 2 500 000 Daltons ;
- l'acide β-(1,3)-D-polyglucuronique peracétylé ayant des masses molaires comprises entre 600 et 3 500 000 Daltons; et
- l'acide β-(1,3)-D-polyglucuronique, sulfaté en positions C2 et C4 ayant des masses molaires comprises entre 800 et 4 000 000 Daltons ;
comme agent raffermissant.

3. Utilisation d'une composition cosmétique comprenant, à titre de principe actif, un ou plusieurs β-(1,3)-glucuronane ou β-(1,3)-glucoglucuronane choisi parmi :
- l'acide β-(1,3)-D-polyglucuronique ayant des masses molaires comprises entre 500 et 2 500 000 Daltons ;
- l'acide β-(1,3)-D-polyglucuronique peracétylé ayant des masses molaires comprises entre 600 et 3 500 000 Daltons; et
- l'acide β-(1,3)-D-polyglucuronique, sulfaté en positions C2 et C4 ayant des masses molaires comprises entre 800 et 4 000 000 Daltons ;
comme composition amincissante.

4. Utilisation d'une composition cosmétique comprenant, à titre de principe actif, un ou plusieurs β-(1,3)-glucuronane ou β-(1,3)-glucoglucuronane choisi parmi :
- l'acide β-(1,3)-D-polyglucuronique ayant des masses molaires comprises entre 500 et 2 500 000 Daltons ;
- l'acide β-(1,3)-D-polyglucuronique peracétylé ayant des masses molaires comprises entre 600 et 3 500 000 Daltons; et
- l'acide β-(1,3)-D-polyglucuronique, sulfaté en positions C2 et C4 ayant des masses molaires comprises entre 800 et 4 000 000 Daltons ;
comme composition raffermissante.

## Patentansprüche

1. Verwendung von β-(1,3)-Glucuronan oder β-(1,3)-Glucoglucuronan, ausgewählt aus:
- β-(1,3)-D-Polyglucuronsäure mit Molmassen im Bereich zwischen 500 und 2 500 000 Dalton;
- peracetylierte β-(1,3)-D-Polyglucuronsäure mit Molmassen im Bereich zwischen 600 und 3 500 000 Dalton; und
- β-(1,3)-D-Polyglucuronsäure, sulfatiert an den Positionen C2 und C4, mit Molmassen im Bereich zwischen 800 und 4 000 000 Dalton;
als Verschlankungsmittel.

2. Verwendung von β-(1,3)-Glucuronan oder β-(1,3)-Glucoglucuronan, ausgewählt aus:
- β-(1,3)-D-Polyglucuronsäure mit Molmassen im Bereich zwischen 500 und 2 500 000 Dalton;
- peracetylierte β-(1,3)-D-Polyglucuronsäure mit Molmassen im Bereich zwischen 600 und 3 500 000 Dalton; und
- β-(1,3)-D-Polyglucuronsäure, sulfatiert an den Positionen C2 und C4, mit Molmassen im Bereich zwischen 800 und 4 000 000 Dalton;
als Straffungsmittel.

3. Verwendung einer kosmetischen Zusammensetzung, umfassend, als Wirkstoff, ein oder mehrere β-(1,3)-Glucuronan oder β-(1,3)-Glucoglucuronan, ausgewählt aus:
- β-(1,3)-D-Polyglucuronsäure mit Molmassen im Bereich zwischen 500 und 2 500 000 Dalton;
- peracetylierte β-(1,3)-D-Polyglucuronsäure mit Molmassen im Bereich zwischen 600 und 3 500 000 Dalton; und
- β-(1,3)-D-Polyglucuronsäure, sulfatiert an den Positionen C2 und C4, mit Molmassen im Bereich zwischen 800 und 4 000 000 Dalton;
als Verschlankungszusammensetzung.

4. Verwendung einer kosmetischen Zusammensetzung, umfassend, als Wirkstoff, ein oder mehrere β-(1,3)-Glucuronan oder β-(1,3)-Glucoglucuronan, ausgewählt aus:
- β-(1,3)-D-Polyglucuronsäure mit Molmassen im Bereich zwischen 500 und 2 500 000 Dalton;
- peracetylierte β-(1,3)-D-Polyglucuronsäure mit Molmassen im Bereich zwischen 600 und 3 500 000 Dalton; und
- β-(1,3)-D-Polyglucuronsäure, sulfatiert an den Positionen C2 und C4, mit Molmassen im Bereich zwischen 800 und 4 000 000 Dalton;
als Straffungszusammensetzung.

## Claims

1. A use of β-(1,3)-glucuronan or β-(1,3)-glucoglucuronan selected from:
- the β-(1,3)-D-polyglucuronic acid having molar masses comprised between 500 and 2,500,000 Daltons;
- the peracetylated β-(1,3)-D-polyglucuronic acid having molar masses comprised between 600 and 3,500,000 Daltons; and
- the β-(1,3)-D-polyglucuronic acid, sulphated in the C2 and C4 positions having molar masses comprised between 800 and 4,000,000 Daltons; as a slimming agent.

2. The use of β-(1,3)-glucuronan or β-(1,3)-glucoglucuronan selected from:
- the β-(1,3)-D-polyglucuronic acid having molar masses comprised between 500 and 2,500,000 Daltons;
- the peracetylated β-(1,3)-D-polyglucuronic acid having molar masses comprised between 600 and 3,500,000 Daltons; and
- the β-(1,3)-D-polyglucuronic acid, sulphated in the C2 and C4 positions having molar masses comprised between 800 and 4,000,000 Daltons; as a firming agent.

3. The use of a cosmetic composition comprising, as an active ingredient, one or more β-(1,3)-glucuronan(s) or β-(1,3)-glucoglucuronan(s) selected from:
- the β-(1,3)-D-polyglucuronic acid having molar masses comprised between 500 and 2,500,000 Daltons;
- the peracetylated β-(1,3)-D-polyglucuronic acid having molar masses comprised between 600 and 3,500,000 Daltons; and
- the β-(1,3)-D-polyglucuronic acid, sulphated in the C2 and C4 positions having molar masses comprised between 800 and 4,000,000 Daltons; as a slimming composition.

4. The use of a cosmetic composition comprising, as an active ingredient, one or more β-(1,3)-glucuronan(s) or β-(1,3)-glucoglucuronan(s) selected from:
- the β-(1,3)-D-polyglucuronic acid having molar masses comprised between 500 and 2,500,000 Daltons;
- the peracetylated β-(1,3)-D-polyglucuronic acid having molar masses comprised between 600 and 3,500,000 Daltons; and
- the β-(1,3)-D-polyglucuronic acid, sulphated in the C2 and C4 positions having molar masses comprised between 800 and 4,000,000 Daltons; as a firming composition.
